# EUROPEAN PATENT APPLICATION

(11) **EP 1 792 917 A1**
(43) Date of publication of application: **06.06.2007**
(21) Application number: 05768868.1
(22) Date of filing: 08.08.2005
(51) Int. Cl.: C07K 14/765, A61K 38/16, A61P 7/08

(54) **SURFACE-MODIFIED SERUM ALBUMIN-METAL PORPHYRIN COMPOSITE, AND OXYGEN INFUSION CONTAINING THE SAME**

(30) Priority: 09.08.2004 JP 2004232519
(71) Applicant: Tsuchida, Eishun, Tokyo 175-0053 (JP)
(72) Inventor: TSUCHIDA, Eishun, Tokyo 175-0053 (JP); KOMATSU, Teruyuki, Tachikawa-shi, Tokyo (JP); HUANG, Yubin, Tokyo (JP)
(74) Representative: Kröncke, Rolf
(86) International application number: PCT/JP2005/014533
(87) International publication number: WO 2006/016561

(57) **Abstract**

A surface-modified serum albumin-metal porphyrin composite that contains serum albumin which clathrates a metal porphyrin and to which a polyoxyethylene group is covalently bonded to the molecular surface of the serum albumin.

## Description

### Technical Field

The present invention relates to a surface-modified serum albumin-metal porphyrin composite obtained by covalently bonding a polyoxyethylene group to a molecular surface of serum albumin clathrating a metal porphyrin, and an oxygen infusion solution (artificial oxygen transporter) containing the same.

### Background Art

Heme, a prosthetic group of hemoglobin and myoglobin which play roles of transporting and storing oxygen in a living body, that is, iron(II) porphyrin, reversibly binds and releases molecular oxygen in response to oxygen partial pressure. Studies intending to reproduce the same oxygen adsorption/desorption ability as that of such natural heme by synthetic iron(II) porphyrins have been reported since 1970's, J. P. Collman, Acc. Chem. Res., 10, 265 (1977), and F. Basolo, B. M. Hoffman, J. A. Ibers, Acc. Chem. Res., 8, 384 (1975) or the like, are representative ones as study examples in an early period, and latest trends in development are also described in Momentau et al., Chem. Rev., 110, 7690 (1994), and J. P. Collman, Chem. Rev., 104, 561 (2004) or the like.

As an iron(II) porphyrin which can form a stable oxygen complex under a room temperature condition, 5, 10, 15, 20-tetrakis (α, α, α, α-o-pivaloylaminophenyl)porphyrin iron(II) complex (hereinafter referred to as FeTpivPP complex) (J. P. Collman, et al., J. Am. Chem. Soc., 97, 1427 (1975)) is known. The FeTpivPP complex, if an axial base, for example, 1-alkyl imidazole, and 1-alkyl-2-methyl imidazole or the like coexists therewith, reversibly binds and release molecular oxygen in an organic solvent such as benzene, toluene, dichloromethane, tetrahydrofuran, and N, N-dimethylformamide at room temperature.

The reversible oxygen coordination activity can be exhibited only when the central iron of the iron porphyrin is in a divalent state, and the oxygen coordination activity is completely lost if the central iron is oxidized to provide an iron(III) complex. Generally, an oxidation reaction of the central iron(II) is accelerated in an aqueous solution, and thus the stability of an oxygen complex to be obtained is significantly low. A research group including the present inventors has overcome this difficulty by embedding the FeTpivPP complex in a bilayer membrane vesicles consisting of phospholipids, and succeeded in exerting the similar oxygen adsorption/desorption function even under physiological conditions (aqueous phase system, pH 7.4, 37°C) (for example, E. Tsuchida et al., J. Chem. Soc., Dalton Trans., 1984, 1147 (1984), and E. Tsuchida et al., J. Chem. Soc., Perkin Trans. 2, 1995, 747 (1995) or the like). This technology of clathrating an iron(II) porphyrin in a phospholipid vesicle not only enables the iron(II) porphyrin to be uniformly dissolved in water, but also has an effect of extending stability of an oxygen complex by providing a micro hydrophobic space at the vicinity of the oxygen coordination site. However, addition of excess moles of axial base molecules from the outside as in the case in an organic solution was indispensable in order that the FeTpivPP complex reversibly binds and releases oxygen. As is well known, some imidazole derivatives employed widely as an axial base has a pharmacological action, and in many cases, intravital toxicity is high. Moreover, when using phospholipid vesicles, an imidazole derivative, made to coexist excessively, can become factor of making its configuration unstable. A method for reducing an added amount of this axial base to the minimum is nothing but introduction of the imidazole derivative into the molecule by a covalent bond.

The research group including the present inventors have considered that a stable oxygen transporter can be provided, without any addition of an axial base from the outside, if an alkylimidazole, for example, as a substituent, is covalently bonded to the molecule of iron(II) porphyrin, and already synthesized a FeTpivPP analogue having a substituent at the 2-position of the porphyrin ring and demonstrated a reversible adsorption/desorption reaction of oxygen on a system in which the analogue is clathrated in a phospholipid vesicle or human serum albumin (Jpn. Pat. Appln. KOKAI Publication No. 1994-271577), Jpn. Pat. Appln. KOKAI Publication No. 1996-301873, and Jpn. Pat. Appln. KOKAI Publication No. 2003-040893). Especially, an albumin-iron(II) porphyrin composite which has been obtained by making this intramolecular base-binding type FepivPP clathrated in human serum albumin has been demonstrated to act as a substitute for red blood cells which have high safety and are capable of transporting oxygen in a living body (E. Tsuchida et al., Bioconjugate Chem. 11, 46 (2000)). Moreover, a side effect such as hypertension found in a molecular hemoglobin preparation is not recognized at all (E. Tsuchida et al., J. Biomed. Mater. Res. 64A, 257 (2003)).

On the other hand, since a clathration driving force of iron(II) porphyrin in this albumin-iron(II) porphyrin composite system is hydrophobic interaction, that is, non-covalent bond, there is also a possibility that the metal porphyrin functioning as an oxygen binding site is dissociated from the hydrophobic site. When an aqueous solution of the above albumin-metal porphyrin composite is administered into the body as an artificial oxygen transporter, for example, as a substitute for red blood cells, it is desirable that such an aqueous solution remains in blood, and plays a role of the oxygen transporter for a certain period. Indeed, when the albumin-metal porphyrin composite is administered into the blood, the metal porphyrin is dissociated from albumin. That is, for realizing a longer half-life in the blood, the present situation is that molecular design and synthesis in which the metal porphyrin is hard to be dissociated from albumin has been longed for.

### Disclosure of Invention

Therefore, an object of the present invention is to solve the problem of the above conventional technology, and provide an albumin-metal porphyrin composite which can form a stable oxygen complex over a longer period and act effectively as an oxygen infusion solution in a living body.

As a result of intensive studies on a molecular design and function expression of a structure in which a metal porphyrin complex is hard to be dissociated from albumin, the present inventors have accomplished the present invention based on the finding that as compared with a conventional serum albumin-metal porphyrin composite in which the metal porphyrin is merely clathrated in the hydrophobic region of albumin, a remaining rate of the metal porphyrin complex inside albumin is high, and as a result, a new oxygen infusion solution extending a half-life in blood can be provided when a surface-modified serum albumin-metal porphyrin composite is prepared by covalently bonding a polyoxyethylene group to the molecular surface of serum albumin clathrating a metal porphyrin.

Thus, according to a first aspect of the present invention, a surface-modified serum albumin-metal porphyrin composite is provided which comprises serum albumin clathrating a metal porphyrin, and in which a polyoxyethylene group is covalently bonded to the molecular surface of the serum albumin.

Moreover, according to a second aspect of the present invention, an oxygen infusion solution (artificial oxygen transporter) containing the surface-modified serum albumin-metal porphyrin composite is provided.

### Best Mode for Carrying Out the Invention

A surface-modified serum albumin-metal porphyrin composite according to the present invention contains serum albumin clathrating a metal porphyrin, and a polyoxyethylene group is covalently bonded to the molecular surface of the serum albumin. Preferably, the polyoxyethylene group is introduced by a thioether bond, and is introduced in particular by the thioether by converting the amino group of lysine residue existing on the molecular surface of serum albumin into a thiolate group with iminothiolane, and by reacting a polyoxyethylene having a terminal maleimido group with the thiolate group.

The metal porphyrin to be clathrated is preferably represented by a formula [I], [II], [III], [IV], or [V] :

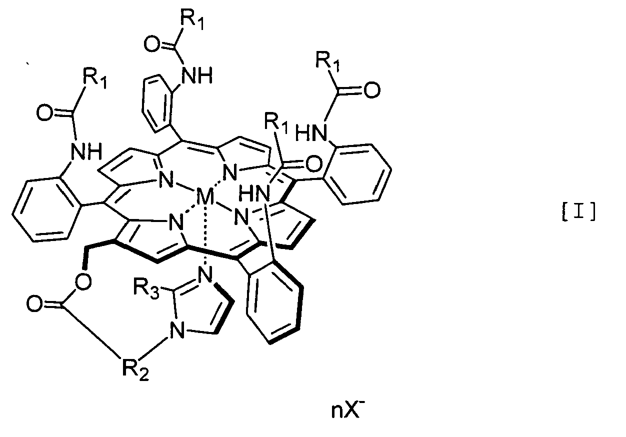

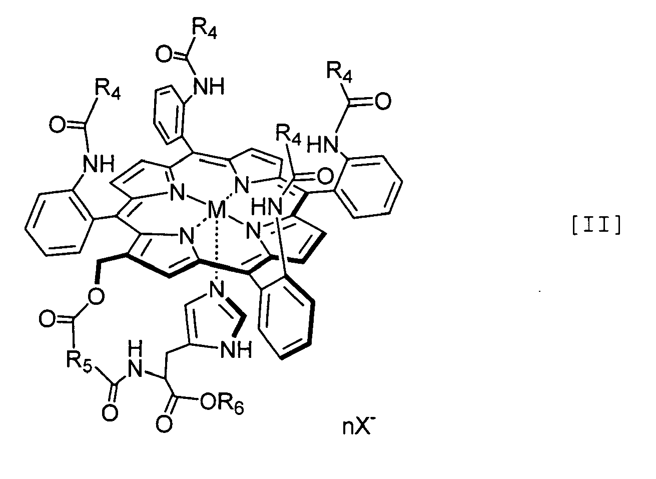

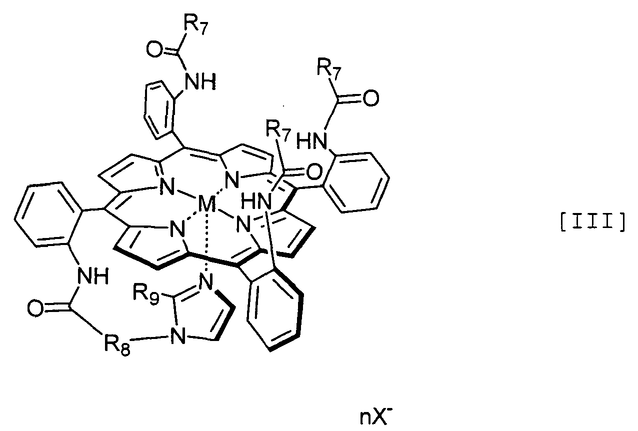

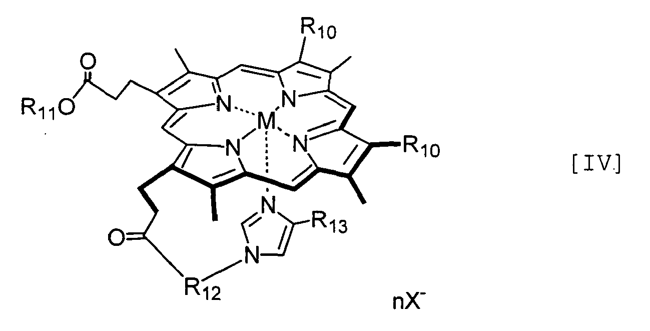

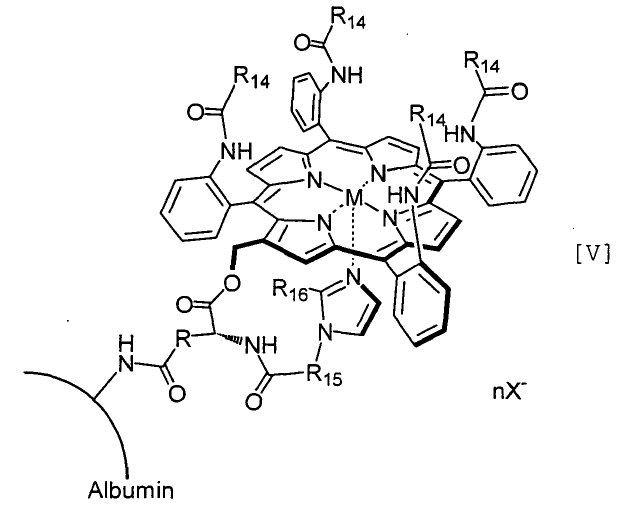

The metal porphyrin represented by the formula [I] is described in Jpn. Pat. Appln. KOKAI Publication No. 1994-271577 and Jpn. Pat. Appln. KOKAI Publication No. 2003-40893. In the formula [I], R₁ is a straight chain or an alicyclic hydrocarbon group which may have a substituent. It is preferable that R₁ is a straight chain or alicyclic hydrocarbon group having a substituent at the 1-position. Examples of the straight chain or alicyclic hydrocarbon groups include a 1, 1-disubstituted C₁ - C₁₀ alkane group, a 1-substituted cyclopropyl group, a 1-substituted cyclopentyl group, a 1-substituted cyclohexyl group, a 2-substituted norbornyl group (the substituent in these groups is a methyl group, an alkylamido group (R'CONH-), an alkylester group (R'OOC-) or an alkyl ether group

(R'O-)), 1-methyl-2-clyclohexenyl group, or 1-adamantyl group or the like. Here, a C₁ - C₆ alkyl group is preferable as an alkyl group represented by R'.

In the formula [I], R₂ is an alkylene group, preferably, a C₁ - C₁₀ alkylene group.

In the formula [I], R₃ is a group allowing the coordination of the imidazolyl group to the central transition metal ion M (ion of a transition metal belonging to the fourth or fifth period of the periodic table). Examples of such R₃ include a hydrogen atom or a methyl group, an ethyl group or a propyl group. X- denotes a halide ion such as a chloride ion or a bromide ion, and n, which denotes the number of X⁻, is a number obtained by deducting 2 from the valence number of the transition metal ion M.

The metal porphyrin represented by the formula [II] is described in Jpn. Pat. Appln. KOKAI Publication No. 2002-128781. In the formula [II], R₄ is a straight chain or an alicyclic hydrocarbon group which may have a substituent. It is preferable that R₄ is a straight chain or alicyclic hydrocarbon group having a substituent at the 1-position. Examples of the straight chain or alicyclic hydrocarbon groups include a 1, 1-disubstituted C₁ - C₁₀ alkane group, a 1-substituted cyclopropyl group, a 1-substituted cyclopentyl group, a 1-substituted cyclohexyl group, a 2-substituted norbornyl group (the substituent in these groups is a methyl group, an alkylamido group (R'CONH-), an alkylester group (R'OOC-) or an alkylether group (R'O-)), 1-methyl-2-clyclohexenyl group, or 1-adamantyl group or the like. Here, a C₁ - C₆ alkyl group is preferable as an alkyl group represented by R'.

In the formula [II], R₅ is an alkylene group, preferably a C₁ - C₆ alkylene group.

In the formula [II], R₆ is an alkyl group, preferably a C₁ - C₆ alkyl group.

In the formula [II], M is a central transition metal ion (ion of a transition metal belonging to the fourth or fifth period of the periodic table). X⁻ denotes a halide ion such as a chloride ion or a bromide ion, and n, which denotes the number of X⁻, is a number obtained by deducting 2 from the valence number of the transition metal ion M.

The metal porphyrin represented by the formula [III] is described in Japanese Patent Application. No. 2004-190189 specification. In the formula [III], R₇ is a straight chain or an alicyclic hydrocarbon group which may have a substituent. It is preferable that R₇ is a straight chain or alicyclic hydrocarbon group having a substituent at the 1-position. Examples of the straight chain or alicyclic hydrocarbon groups include a 1, 1-disubstituted C₁ - C₁₀ alkane group, a 1-substituted cyclopropyl group, a 1-substituted cyclopentyl group, a 1-substituted cyclohexyl group, a 2-substituted norbornyl group (the substituent in these groups is a methyl group, an alkylamido group (R'CONH-), an alkylester group (R'OOC-) or an alkylether group (R'O-)), 1-methyl-2-clyclohexenyl group, or 1-adamantyl group or the like. Here, a C₁ - C₆ alkyl group is preferable as an alkyl group represented by R'.

In the formula [III], R₈ is an alkylene group, preferably, a C₁ - C₆ alkylene group.

In the formula [III], R₉ is a group allowing the coordination of the imidazolyl group to the central transition metal ion M (ion of a transition metal belonging to the fourth or fifth period of the periodic table), preferably a hydrogen atom, a methyl group, an ethyl group, or a propyl group, and R is a methylene group or an ethylene group. X- denotes a halide ion such as a chloride ion or a bromide ion, and n, which denotes the number of X⁻, is a number obtained by deducting 2 from the valence number of the transition metal ion M.

The metal porphyrin represented by the formula [III] can be synthesized, for example, as follows. A meso-tetrakis(o-aminophenyl)porphyrin is dissolved in a suitable organic solvent (for example, chloroform, tetrahydrofuran or the like), trityl bromide and triethylamine are added thereto, and after further agitation for 10 minutes to 1 hour, the solvent is removed under reduced pressure. The toluene solution of porphyrin obtained is charged in a container in which activated alumina and heptane are contained, and agitated at 90°C under a light-shielded condition for 12 to 24 hours. The alumina is filtered off with a glass filter, and meso-tri (α, α, α -o-aminophenyl) - ( *β* -o- (N-triphenylmethyl)aminophenyl)porphyrin is fractionally purified by a silica gel column. The obtained porphyrin and a solution of a suitable base (pyridine, 4-dimethylaminopyridine, triethylamine or the like) dissolved in a suitable organic solvent (for example, chloroform, tetrahydrofuran or the like) are added dropwise to an acid chloride represented by a formula R₇COCl (wherein R₇ is as defined above) and agitated at room temperature for 2 to 15 hours under nitrogen atmosphere. After the solvent is removed under reduced pressure, chloroform and water are added, and after the aqueous layer is washed with chloroform, the organic layer is recovered, and the solvent is removed under reduced pressure. The obtained residue is fractionally purified by a silica gel column, and dried in vaccuo, affording meso-tri(α, α, α -o- (substituted amido) phenyl) - ( β -o- (N-triphenylmethyl)aminophenyl) porphyrin as a solid. The obtained porphyrin and a solution of a suitable base (pyridine, 4-dimethylaminopyridine, triethylamine or the like) dissolved in a suitable organic solvent (for example, chloroform, tetrahydrofuran or the like) are added dropwise to an acid chloride of an ω-imidazolylcarboxylic acid represented by a formula [VI] below (in the formula [VI], R₈ to R₉ is as defined above) and agitated under nitrogen atmosphere at room temperature for 2 to 15 hours.

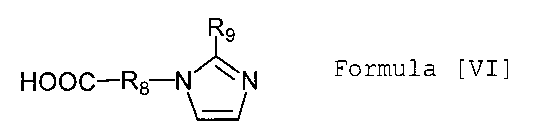

Next, after the solvent is removed under reduced pressure, the chloroform layer is washed with water, and the solvent is removed under reduced pressure. The obtained residue is fractionally purified by the silica gel column and dried under vacuum. Thus, the porphyrin compound represented by the formula [III] can be obtained.

Introduction of the central metal into the thus obtained porphyrin compound is achieved by a general method described in, for example, The Porphyrin, D. Dolphin ed., Academic Press Inc., 1978 or the like, affording the corresponding porphyrin metal complex.

The metal porphyrin represented by the formula [IV] is described in the above patent document 2 (Jpn. Pat. Appln. KOKAI Publication No. 1996-301873). In the formula [IV], R₁₀ is a hydrocarbon group. R₁₀ is preferably a hydrogen atom, a vinyl group, an ethyl group, a formyl group, or an acetyl group.

In the formula [IV], R₁₁ is an alkyl group, preferably a C₁ - C₁₀ alkyl group.

In the formula [IV], R₁₂ is an alkylene group, preferably a C₁ - C₁₀ alkylene group.

In the formula [IV], R₁₃ is a group allowing the coordination of the imidazolyl group to the central transition metal ion M (ion of a transition metal ion belonging to the fourth or fifth period of the periodic table). Examples of such R₁₃ include a hydrogen atom, a methyl group, an ethyl group, or a propyl group. X- denotes a halide ion such as a chloride ion or a bromide ion, and n, which denotes the number of X⁻, is a number obtained by deducting 2 from the valence number of the transition metal ion M.

The metal porphyrin represented by the formula [V] is described in Japanese Patent Application. No. 2004-190190 specification. In the formula [V], R is a methylene group or an ethylene group. R₁₄ is a straight chain or an alicyclic hydrocarbon group which may have a substituent. It is preferable that R₁₄ is a straight chain or alicyclic hydrocarbon group having a substituent at the 1-position. Examples of the straight chain or alicyclic hydrocarbon groups include a 1, 1-disubstituted C₁ - C₁₀ alkane group, a 1-substituted cyclopropyl group, a 1-substituted cyclopentyl group, a 1-substituted cyclohexyl group, a 2-substituted norbornyl group (the substituent in these groups is a methyl group, an alkylamido group (R'CONH-), an alkylester group (R'OOC-) or an alkylether group (R'O-)), 1-methyl-2-clyclohexenyl group, or 1-adamantyl group or the like. Here, a C₁ - C₆ alkyl group is preferable as an alkyl group represented by R'.

In the formula [V], R₁₅ is an alkylene group, preferably a C₁ - C₁₀ alkylene group.

In the formula [V], R₁₆ is a group allowing the coordination of the imidazolyl group to the central transition metal ion M (ion of a transition metal belonging to the fourth or fifth period of the periodic table), preferably a hydrogen atom, a methyl group, an ethyl group, or a propyl group. X- denotes a halide ion such as a chloride ion or a bromide ion, and n, which denotes the number of X⁻, is a number obtained by deducting 2 from the valence number of the transition metal ion M.

Metal porphyrin shown by the formula [V] can be synthesized by using, for example, 2-hydroxymethyl-5,10, 15, 20-tetrakis (α, α, α, α-o-substituted amidophenyl)porphyrin synthesized by using a method described in Tsuchida et al., J. Chem. Soc. Perkin Trans 2, 1995, 747 (1995), as a starting material. Specifically, an aspartic acid or glutamic acid derivative represented by a formula [A]: R"OOCRC(NH-Fmoc)COOH (where R is as defined above, Fmoc is 9-fluorenylmethyloxycarbonyl, and R" is, for example, t-butyl) is dissolved in a suitable dried solvent (for example, dichloromethane, benzene, dimethylformamide or the like), dicyclohexylcarbodiimide (DCC) is added as a condensing agent, and agitated at room temperature for 1 to 12 hours. Since white precipitate is formed as the reaction proceeds, after the reaction solution is cooled in an ice bath for 2 to 30 minutes, the precipitated N,N'-dicyclohexylurea (DCU) is removed by filtration. A 2-hydroxymethyl-5,10,15,20-tetrakis(α, α, α, α-o-substituted amidophenyl)porphyrin is added to the filtrate and agitated under a light-shielded condition for 1 to 12 hours. The progression of the reaction is traced by thin layer chromatography (TLC), and a suitable base (pyridine, dimethylaminopyridine, triethylamine or the like) is added, if necessary. The solvent is removed under reduced pressure, the residue is dissolved in benzene, and the precipitated DCU is removed by filtration again. After the solvent is removed under reduced pressure, cold hexane is added to precipitate the porphyrin. The obtained mixture is purified by silica gel column chromatography, affording 2-(N-Fmoc-t-butoxycarbonyl-aminoacyl)methyl-5,10,15,20-tetrakis (α, *α ,* α, α -o-substituted amidophenyl)porphyrin. Needless to say, the aminoacyl here is asparagyl or glutamyl (the same applies hereinafter). The obtained porphyrin is dissolved in dimethylformamide, piperidine is added and agitated under a light-shielded condition at room temperature for 6 to 24 hours. After dimethylformamide and piperidine are removed under reduced pressure, and the residue is purified by silica gel column chromatography, affording 2-(t-butoxycarbonyl-aminoacyl)methyl-5, 10, 15, 20-tetrakis (α, α, α, α -o-substituted amidophenyl)porphyrin. Next, a hydrochloride of an ω-imidazolylalkanoic acid represented by a formula [VII] below (in the formula [VII], R₁₅ and R₁₆ are as defined above) is dissolved in distilled dimethylformamide, DCC and a suitable base (for example, pyridine, dimethylaminopyridine, triethylamine or the like) are added, and agitated at room temperature for 30 minutes to 4 hours.

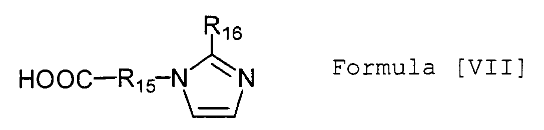

The DCU formed according to the progression of the reaction is removed by filtration, 2-(t-butoxycarbonyl-aminoacyl) methyl-5, 10, 15, 20-tetrakis (α, α, α, α -o-substituted amidophenyl)porphyrin is added to the filtrate, and agitated under a light-shielded condition for 15 minutes to 2 hours. The solvent is removed under reduced pressure, the residue is redissolved in a mixed solvent of dichloromethane and triethylamine, and agitated under a light-shielded condition at room temperature for 6 to 24 hours. After the DCU is removed by filtration, the residue is dissolved in benzene, the precipitates are filtered off and the solvent is removed under reduced pressure. The residue is dissolved in chloroform, washed with pure water and an aqueous solution of sodium hydrogencarbonate, dewatered, and the chloroform is removed, affording 2-(N-(ω-imidazolylalkanoyl)-t-butoxycarbonylaminoacyl) methyl-5, 10, 15, 20-tetrakis (α, α, α, α -o-substituted amidophenyl)porphyrin. Since this compound is decomposed by light and separation operation by a silica gel column, it is employed for the next reaction as obtained.

The thus obtained porphyrin is dissolved in a suitable dried solvent (dichloromethane, chloroform, benzene or the like), trifluoroacetic acid is added, and agitated under a light-shielded condition at room temperature for 1 to 6 hours. The progression of the reaction is traced by TLC, the solvent is removed under reduced pressure, and benzene is added to the residue, affording 2-(N-(ω-imidazolylalkanoyl)-aminoacyl)methyl-5, 10, 15, 20-tetrakis (α, α*,* α, α -o-substituted amidophenyl)porphyrin. Since this compound is decomposed by light and separation operation by silica gel column, it is employed for the next reaction as obtained.

The central metal M is introduced into the obtained porphyrin. The introduction of the central metal M is achieved by a general method described in, for example, The Porphyrin, D. Dolphin ed., 1978, Academic Press. Inc. or the like, affording the corresponding porphyrin metal complex. In general, in the case of an iron complex, a porphyrin iron(III) complex is obtained and in the case of a cobalt complex, a porphyrin cobalt (II) complex is obtained. Specifically, a solution of 2-(N-(ω-imidazolylalkanoyl)-aminoacyl)methyl-5, 10, 15, 20-tetrakis (α, α, α, α-o-substituted amidophenyl)porphyrin in distilled THF is quickly added to iron(II) bromide prepared by electrolytic iron and hydrobromic acid, under a dry argon atmosphere, and reacted at 60 to 80°C for 2 to 24 hours. After the solvent is removed under reduced pressure, the residue is dissolved in chloroform and washed with pure water sufficiently. After dehydration and removal of the solvent, the obtained mixture is fractionally purified by silica gel column chromatography, affording a brown solid 2-(N-(ω-imidazolylalkanoyl)-aminoacyl)methyl-5, 10, 15, 20-tetrakis (α, α , α , α -o-substituted amidophenyl)porphinato iron(III).

This porphyrin is dissolved in a suitable dried solvent (dichloromethane, chloroform, benzene, diethyl ether or the like), and DCC is added and agitated at room temperature for 10 minutes to 2 hour. A dichloromethane solution of N-hydroxysuccinimide is added thereto, and reacted under a light-shielded condition at room temperature for 2 to 24 hours. The progression of the reaction is traced by TLC, the precipitates are filtered off at 0°C, and the solvent is removed under reduced pressure. The residue is dissolved in benzene, DCU is removed again by filtration, and the solvent is removed under reduced pressure, affording the aimed compound 2-(N-(ω-imidazolylalkanoyl)-succinimidyl-aminoacid ester) methyl-5, 10, 15, 20-tetrakis (*α,* α , α , α -o-substituted amidophenyl)porphinato iron(III) is obtained. Here, needless to say, the amino acid ester is alginate or glutamate.

Incidentally, among the above porphyrin metal complexes, in the case of having a form of an iron(III) complex, oxygen binding activity can be imparted when the central metal is reduced from trivalence to bivalence, using a suitable reducing agent (sodium dithionite, ascorbic acid or the like).

In order to bond these porphyrin iron(II) complexes with albumin, firstly, an ethanol solution of a carbonyl complex of the porphyrin iron(II) complex is prepared, which is mixed with an aqueous phosphate buffer solution of, for example, human serum albumin, and agitated slowly at room temperature for 30 minutes to 3 hours. The obtained solution is dialyzed with respect to an aqueous phosphate buffer for 10 to 24 hours, and the ethanol is removed. Thus, the metal porphyrin represented by the formula [V] is obtained. Here, the acyl group remaining after the succinimidyloxy group of porphyrin has been eliminated forms an amide bond with a lysine amino group of the albumin.

The human serum albumin clathrating the metal porphyrin of the present invention may be a recombinant human serum albumin-metal porphyrin complex obtained by an axial coordination bonding of the metal porphyrin to a recombinant human serum albumin in which at least one amino acid, which brings about a bond with the metal porphyrin, is introduced by a genetic recombination technology. Examples of a recombinant human serum albumin-metal porphyrin complex are described in Jpn. Pat. Appln. KOKAI Publication No. 2002-500862. Amino acid to be introduced is preferably histidine, and the introduction position is preferably the subdomain IB. The metal porphyrin in this case is preferably a metal protoporphyrin, a metal dueteroporphyrin, a metal diacetyldueteroporphyrin, a metal mesoporphyrin, a metal diformylporphyrin, a metal tetraphenylporphyrin, and a metal octaethylporphyrin, which do not have an axial base ligand in the molecule.

In the surface-modified serum albumin-metal porphyrin composite of the present invention, the central transition metal ion M is preferably Fe or Co. The atomic valence number of Fe can be positive bivalent or positive trivalent, and the atomic valence number of Co can be positive bivalent.

Moreover, in the surface-modified serum albumin-metal porphyrin composite of the present invention, serum albumin is preferably human serum albumin, bovine serum albumin, recombinant human serum albumin, and albumin multimer.

The average number of polyoxyethylene molecules bonded per albumin molecule is preferably 1 to 15, and the average molecule weight of polyoxyethylene is preferably 1,000 to 20,000.

Since such surface-modified serum albumin-metal porphyrin composite can bind and release oxygen reversibly, not only it surely exerts a function as an oxygen infusion solution, but also the metal porphyrin clathrated inside the albumin has a characteristic that it is hard to be dissociated since the molecular surface of albumin is covered by the polyoxyethylene chain. Namely, even in the case of being administered into a body, the metal porphyrin is never disengaged from albumin in a blood circulating system, and a longer residence time in blood is expected.

As for adaptation of the oxygen infusion solution, besides a resuscitating solution of a bleeding shock (blood substitute for blood transfusion), preoperational blood dilution solution, a filling solution of a extracorporeal circulation circuit such as an artificial heart-lung, a perfusate for a transplanted organ, an oxygen supply solution to an ischaemia region (myocardial infarction, cerebral infarction, respiratory failure or the like), therapeutic agent of chronic anemia, a return flow liquid of liquid ventilation, a sensitizer for tumor therapy, culture solution for a regenerated tissue cell, and furthermore, utilization for a rare blood-type patient, support for a patient refusing blood transfusion because of religious reason, and application for animal medical treatment, are expected.

In addition, when the porphyrin is in the form of a complex with, for example, ion of a metal belonging to the fourth or fifth period of the periodic table, an added value as a catalyst of a redox reaction, an oxidation reduction reaction, an oxygen oxidation reaction or an oxygen addition reaction, is also high. Therefore, apart from as an oxygen infusion, the porphyrin metal complex of the invention has characteristics as a gas adsorbent, a redox catalyst, an oxygen oxidation reaction catalyst, and an oxygenation reaction catalyst.

Although there is no limit to the manufacturing method of the surface-modified serum albumin-metal porphyrin complex of this invention, for example, it is synthesized by the following method.

To a phosphate buffer physiological saline solution (PBS, pH 7.4) of a human serum albumin composite clathrating 2-(N-(8-(2-methylimidazolyl)octanoyloxy))methyl-5, 10, 15, 20-tetrakis (α, α, α, α -o- (1-methylcyclohexanamido)phenyl)porphinato iron(II) (carbon monoxide) complex (albumin concentration: 0.1 to 10 wt%, preferably 0.5 to 5.0 wt%, and iron(II) porphyrin/albumin: 1 to 8 (mol/mol)) synthesized by a method described in Jpn. Pat. Appln. KOKAI Publication No. 2003-040893, for example, iminothiolane (manufactured by Pierce Chemical Co., Ltd., iminothiolane/albumin: 3 to 40 (mol/mol), preferably 10 to 30) was added, and agitated at room temperature for 1 to 12 hours, preferably for 2 to 6 hours. Subsequently, a polyoxyethylene terminated with maleimide at one end and with methyl at the other end, for example, Sunbright Memarl 50-H (Mw: 5000, manufactured by Nippon Oil & Fats Co., Ltd., polyoxyethylene/albumin: 3 to 40 (mol/mol), preferably 10 to 30) is added, and reacted for 0.5 to 6 hours, preferably for 1 to 3 hours under the same conditions. For the obtained mixture, concentration and washing were repeated with several liters of a PBS solution, by using an ultrafiltration device (manufactured by Advantech Co., Ltd., UHP-76k, ultrafiltration molecular membrane: 5 kDa). The red-colored surface-modified serum albumin iron(II) porphyrin composite aqueous solution obtained is passed through a sterilizing filter of 0.45 µm (manufactured by Advantech Co., Ltd., DISMIC 25CSO45AS) for the final adjustment.

The iron ion concentration of the surface-modified serum albumin-iron(II) porphyrin composite is measured by using an inductively coupled plasma atomic emission spectrometer (ICP, SPS 7000A, manufactured by Seiko Instruments Co., Ltd.), and can be used as an index of the heme concentration. Moreover, the albumin concentration can be measured by a bromocresol green method (manufactured by a Wako Pure Chemical Co., Ltd., Albumin Test Wako). Furthermore, the molecular weight of the surface-modified serum albumin-iron(II) porphyrin complex can be measured by a matrix assisted laser desorption/ionization mass spectrometry (MALDI-TOFMS) (manufactured by Shimadzu Corporation, KRATOS AXIMA-CFR). From the above analytical results, the number of the polyoxyethylene chains bonded to the albumin surface can be found.

Any one of the surface-modified serum albumin-iron(II) porphyrin complexes rapidly forms a stable oxygen complex when contacted with oxygen. Moreover, these complexes can absorb and desorb oxygen in response to oxygen partial pressure. This oxygen bonding and releasing can be carried out repeatedly and reversibly, and they act as an oxygen adsorbent/desorbent, and an oxygen carrier.

In the case of a metal-coordinating gas other than oxygen, a corresponding coordination complex can be formed (for example, carbon monoxide, nitrogen monoxide, and nitrogen dioxide). From these reasons, especially in the case of iron(II) or cobalt (II) complex, the porphyrin metal complex of the present invention can be used as a redox reaction catalyst in a homogeneous system or a heterogeneous system, in addition to the fact that it functions as an effective oxygen infusion solution.

Their residence time in blood can be determined by administering an aqueous solution of the surface-modified serum albumin-iron(II) porphyrin complex (oxygen complex) to a Wister rat in anesthesia from its tail vein, and thereafter measuring the concentrations of the iron(II) porphyrins contained in blood plasmas obtained with time. A half-life period of the iron(II) porphyrin in blood is 12 to 24 hours, and this is extended by approximately 7 to 34 times compared with the serum albumin-iron(II) porphyrin composite which is not modified by polyoxyethylene.

The present invention will be explained in detail below by way of Examples. It is needless to say that the present invention is not limited to these Examples.

### Example 1

To a phosphate buffer physiological solution (PBS, pH 7.4, 50 mL) of a human serum albumin composite clathrating 2-(N-(8-(2-methylimidazolyl)octanoyloxy))methyl-5,10,15,20-tetrakis (α, α, *α, α -*o*-*(1-methylcyclohexanamido)phenyl)porphinato iron(II) (carbon-monoxide) complex (albumin concentration: 1 wt%, iron(II) porphyrin/albumin: 4 (mol/mol)) synthesized by a method described in Jpn. Pat. Appln. KOKAI Publication No. 2003-040893, 20 mg of iminothiolane (manufactured by Pierce Chemical Co., Ltd., iminothiolane/albumin: 20 (mol/mol)) was added, and slowly agitated at room temperature for four hours. Subsequently, a polyoxyethylene terminated with maleimide at one end and with methyl at the other end (Sun Bright Memarl 50-H, Mw: 5,000, manufactured by Nippon Oil & Fats Co., Ltd. 0.6 g) was added and reaction was carried out for two hours under the same conditions. The mixture thus obtained was concentrated and washed with 1 L of a PBS solution repeatedly by using an ultrafiltration device (UHP-76K, ultrafiltration molecular membrane: 5 kDa, manufactured by Advantech Co., Ltd.), and was finally concentrated to 10 mL. The aqueous solution of the red-colored surface-modified serum albumin-iron(II) porphyrin composite thus obtained was passed through a sterilizing filter of 0.45 µm (DISMIC 25CS045AS, manufactured by Advantech Co., Ltd.) for the final adjustment.

The iron-ion concentration of the red-colored surface-modified serum albumin-iron(II) porphyrin complex thus obtained was measured by means of an inductively coupled plasma atomic emission spectrometer (ICP, SPS 7000A, manufactured by Seiko Instruments Co., Ltd.), and was used as an index for the heme concentration. Also, the albumin concentration was measured by a bromocresol green method (Albumin Test Wako, manufactured by Wako Pure Chemical Co., Ltd.). Furthermore, the molecular weight of the surface-modified serum albumin-iron(II) porphyrin complex was measured by a matrix assisted laser desorption/ionization mass spectrometer (MALDI-TOFMS) (KRATOS AXIMA-CFR, manufactured by Shimadzu Corporation). As a result of the above experiments, it has been determined that the concentration of the iron(II) porphyrin is 3.0 mM, the albumin concentration is 4.9 mM, the molecular weight is 101,516, the number of the polyoxyethylene chains bound to the surface of the albumin is 6 in average.

### Example 2

A surface-modified serum albumin-iron (II) porphyrin composite aqueous solution was obtained according to the same procedures as in Example 1, except that a phosphate buffer aqueous solution (PBS, pH 7.4, 50 mL) of a human serum albumin composite clathrating 2-(4-(o-methyl-L-histidylcarbonyl)butanoyloxy)methyl-5,10,15,20-tetrakis (α, α, α, α -o-pivalamidophenyl) porphinato iron(II) (carbon monoxide) complex (albumin concentration: 1 wt%; iron(II) porphyrin/albumin: 8 (mol/mol) synthesized by a method described in Jpn. Pat. Appln. KOKAI Publication No. 2002-128781 was used instead of the human serum albumin composite chlathrating 2-(N-(8-(2-methylimidazolyl)octanoyloxy))methyl-5,10,15,20-tetrakis (α, α, α, α -o- (1-methylcyclohexaneamido)phenyl))porphinato iron(II) (carbon monoxide) complex used in Example 1. The number of the polyoxyethylene chain bonded to the albumin surface was 6 in average.

### Example 3

A surface-modified serum albumin-iron(II) porphyrin composite aqueous solution was obtained according to the same procedures as in Example 1, except that a phosphate buffer solution (PBS, pH 7.4, 50 mL) of 3,18-divinyl-8-(3-ethoxycarbonyl)ethyl-12-(N-imidazolylpropylamido)-9-oxymethyl)carbonyl)ethyl-2,7,13,17-tetramethylporphinato iron(II) (carbon monoxide) complex synthesized by a method described in a patent document 2 (Jpn. Pat. Appln. KOKAI Publication No. 1996-301873) (albumin concentration: 1 wt%, iron(II) porphyrin/albumin: 1 [mol/mol]) was used instead of the human serum albumin composite clathrating 2-N-(8-(2-methylimidazolyl)octanoyloxy))methyl-5,10,15,20-tetrakis (α, α, α, α -o- (1-methylcyclohexaneamido)phenyl)porphinato iron(II) (carbon monoxide) complex used in Example 1, and iminothiolane/albumin: 15 (mol/mol) was used., The number of the polyoxyethylene chains bonded to the albumin surface was 5 in average.

### Example 4

The surface-modified serum albumin-iron (II) porphyrin complex (carbon monoxide complex) aqueous solution (4 mL) prepared in Example 1 was put into a 1 cm quartz cell for spectroscope, and, light was irradiated onto the porphyrin iron(II) complex coupled albumin aqueous solution (10 minutes) by using a halogen lamp (500 W), while being cooled in ice water and passing (flowing) oxygen therethrough. Thereafter, ultraviolet-visible absorption spectrum measurement of the obtained aqueous solution was carried out. After confirmation of the formation of an oxygen complex, nitrogen was passed therethrough to effect deoxygenation, preparing the deoxy form. It was found that the absorption spectrum in a nitrogen atmosphere of the porphyrin iron(II) complex coupled albumin has λmax at 444, 540 and 566 nm, and that the iron of the clathrated iron(II) porphyrin forms Fe (II) high spin five-coordinate complex with one intramolecular axial base coordinated thereto. When oxygen was passed through this liquid dispersion, the λmax of the visible absorption spectrum was shifted to 428 and 551 nm, indicating that it has become an oxygenated complex. The visible absorption spectrum reversibly changed from an oxygenated form spectrum to a deoxy form spectrum by blowing nitrogen gas into this oxygenated complex solution for one minute, confirming that, adsorption/desorption of oxygen reversibly takes place. In addition, it was possible to carry out the adsorption/desorption of oxygen successively by repeating an operation of blowing oxygen and then blowing nitrogen.

### Example 5

The aqueous solution (3.8 ml) of the surface modified serum albumin-iron(II) porphyrin complex (oxygen complex) prepared in Example 4 was administered to a Wister rat (male, body weight: about 300 g), anesthetized with sevoflurane, from its tail vein at a rate of 1 ml per minute (dosage amount equivalent to about 20% of the entire blood amount). After the administration, the blood was sampled during a course of time through the femoral artery. The blood obtained was centrifuged (3,000 rpm, 10 minutes at 4°C), and the concentration of the iron(II) porphyrin contained in the blood plasma was measured by the ICP measurement to determine its residence time in blood (the number (n) of the rats used = 5). The half-life period in blood of the iron(II) porphyrin was about 15 hours, which is lengthened to about 20 times in comparison to that of the serum albumin iron(II) porphyrin complex that is not modified with a polyoxyethylene.

As has been described above, a surface-modified serum albumin-metal porphyrin complex according to the present invention has a characteristic that the metal porphyrin clathrated inside it is hard to be dissociated since the surface of the albumin molecule is covered with the polyoxyethylene group. In other words, the oxygen infusion solution containing a surface-modified serum albumin-metal complex according to the present invention can be provided as a preparation that has lengthened residence time in blood, while maintaining an oxygen bonding ability, is useful for practical uses, and in which the metal porphyrin is hard to be dissociated. Furthermore, a surface-modified serum albumin metal porphyrin complex according to the present invention is also useful for applications other than the above mentioned oxygen infusion solution, such as a gas adsorbent, an oxygen adsorbent, a redox catalyst, and an oxygen oxidization reaction catalyst.

## Claims

1. A surface-modified serum albumin-metal porphyrin composite comprising serum albumin which clathrates a metal porphyrin, wherein a polyoxyethylene group is covalently bonded to a molecular surface of the serum albumin.

2. The surface-modified serum albumin-metal porphyrin composite according to claim 1, wherein the polyoxyethylene group is introduced at the molecular surface of the serum albumin by a thioether bond.

3. The surface-modified serum albumin-metal porphyrin composite according to claim 2, wherein the polyoxyethylene group is introduced by converting an amino group of lysine moiety existing on the molecular surface of the serum albumin into a thiolate with iminothiolane, with which thiolate a polyoxyethylene terminated with maleimide at its one end is reacted.

4. The surface-modified serum albumin-metal porphyrin composite according to claim 1, wherein the metal porphyrin is represented by a general formula [I] : 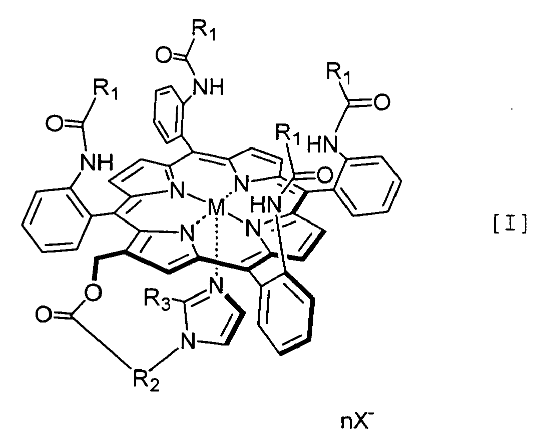 where R₁ is a straight chain or alicyclic hydrocarbon group that may have a substituent, R₂ is an alkylene group, and R₃ is a group allowing the coordination of the imidazolyl group to the central transition metal ion M (ion of a transition metal belonging to the fourth or fifth period of the periodic table).

5. The surface-modified serum albumin-metal porphyrin composite according to claim 4, wherein R₁ is a straight chain or alicyclic hydrocarbon group having a substituent at 1-position, R₂ is a C₁ - C₁₀ alkylene group, and R₃ is a hydrogen atom, a methyl group, an ethyl group, or a propyl group.

6. The surface-modified serum albumin-metal porphyrin composite according to claim 5, wherein R₁ is a 1,1-disubstituted C₁ - C₁₀ alkane group, a 1-substituted cyclopropyl group, a 1-substituted cyclopentyl group, a 1-substituted cyclohexyl group, a 2-substituted norbornyl group (the substituent in these groups is a methyl group, an alkylamido group (R'CONH-), an alkylester group (R'OOC-) or an alkyl ether group (R'O-)), 1-methyl-2-clyclohexenyl group, or 1-adamantyl group.

7. The surface-modified serum albumin-metal porphyrin composite according to claim 1, wherein the metal porphyrin is represented by a general formula [II]: 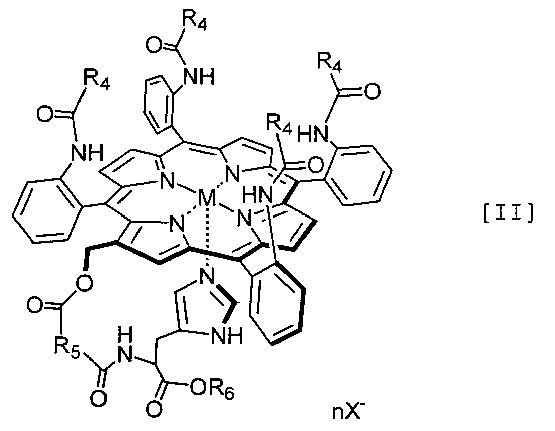 where R₄ is a straight chain or alicyclic hydrocarbon group that may have a substituent, R₅ is an alkylene group, and R₆ is an alkyl group, and M is the central transition metal ion (ion of a transition metal belonging to the fourth or fifth period of the periodic table).

8. The surface-modified serum albumin-metal porphyrin composite according to claim 7, wherein R₄ is a straight chain or alicyclic hydrocarbon group having a substituent at 1-position, R₅ is a C₁ - C₆ alkylene group, and R₆ is a C₁ - C₆ alkyl group.

9. The surface-modified serum albumin-metal porphyrin composite according to claim 8, wherein R₄ is a 1,1-disubstituted C₁ - C₁₀ alkane group, 1-substituted cyclopropyl group, 1-substituted cyclopentyl group, 1-substituted cyclohexyl group, or 2-substituted norbornyl group (the substituent in these groups is a methyl group, an alkylamido group, an alkylester group, or an alkylether group), 1-methyl-2-clyclohexenyl group, or 1-adamantyl group.

10. The surface-modified serum albumin-metal porphyrin composite according to claim 1, wherein the metal porphyrin is represented by a general formula [III]: 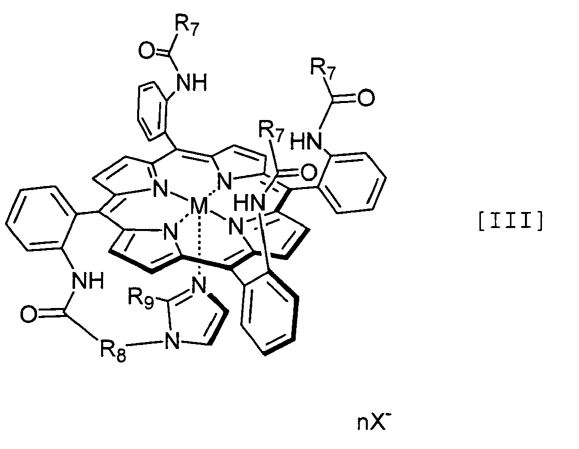 where R₇ is a straight chain or alicyclic hydrocarbon group that may have a substituent, R₈ is an alkylene group, and R₉ is a group allowing coordination of the imidazolyl group to a central transition metal ion M (ion of a transition metal belonging to the fourth or fifth period of the periodic table).

11. The surface-modified serum albumin-metal porphyrin composite according to claim 10, wherein R₇ is a straight chain or alicyclic hydrocarbon group having a substituent at 1-position, R₈ is a C₁ - C₆ alkylene group, and R₉ is a hydrogen atom, a methyl group, an ethyl group or a propyl group.

12. The surface-modified serum albumin-metal porphyrin composite according to claim 11, wherein R₇ is a 1,1-disubstituted C₁ - C₁₀ alkane group, a 1-substituted cyclopropyl group, a 1-substituted cyclopentyl group, a 1-substituted cyclohexyl group, or a 2-substituted norbornyl group (the substituent in these groups is a methyl group, an alkylamido group, an alkylester group, or an alkylether group), 1-methyl-2-clyclohexenyl group, or 1-adamantyl group.

13. The surface-modified serum albumin-metal porphyrin composite according to claim 1, wherein the metal porphyrin is represented by a general formula [IV]: 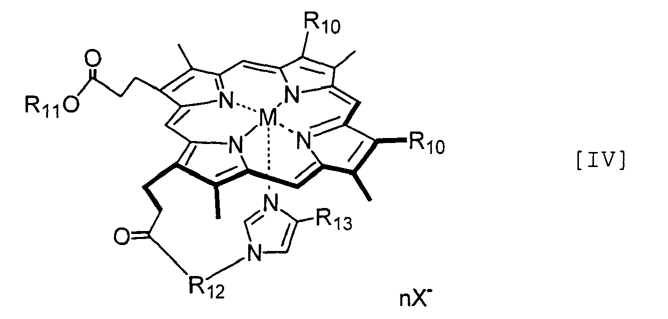 where R₁₀ is a hydrocarbon group, R₁₁ is an alkyl group, R₁₂ is an alkylene group, and R₁₃ is a group allowing the coordination of the imidazolyl group to a central transition metal ion M (ion of a transition metal belonging to the fourth or fifth period of the periodic table).

14. A surface-modified serum albumin-metal porphyrin composite according to claim 13, wherein R₁₀ is a hydrogen atom, a vinyl group, an ethyl group, a formyl group or an acetyl group, and R₁₁ is a C₁ - C₁₀ alkylene group, and R₁₂ is a C₁ - C₁₀ alkylene group, and R₁₃ is hydrogen atom, methyl group, ethyl group or propyl group.

15. The surface-modified serum albumin-metal porphyrin composite according to claim 1, wherein the metal porphyrin is represented by a general formula [V]: 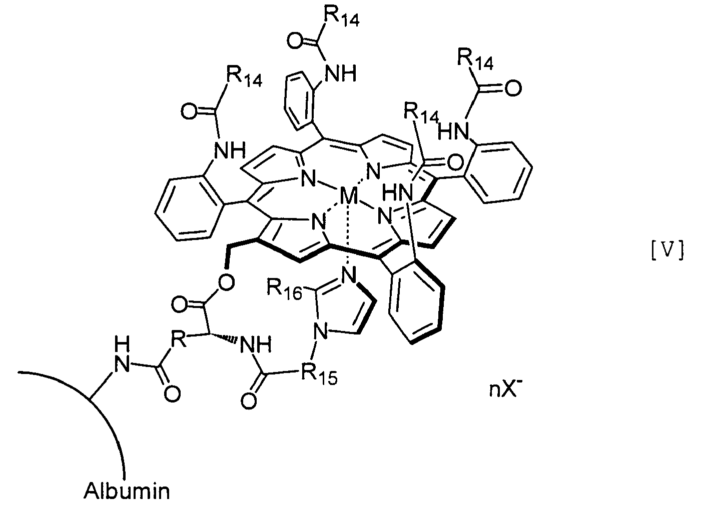 where R₁₄ is a straight chain or alicyclic hydrocarbon group that may have a substituent, R₁₅ is an alkylene group, and R₁₆ is a group allowing the coordination of the imidazolyl group to a central transition metal ion M (ion of a transition metal belonging to the fourth or fifth period of the periodic table), and R is a methylene group or an ethylene group; and is bonded to an amino group which is a lysine moiety of the serum albumin.

16. The surface-modified serum albumin-metal porphyrin composite according to claim 15, wherein R₁₄ is a straight chain or alicyclic hydrocarbon group that may have a substituent at 1-position, R₁₅ is a C₁ - C₁₀ alkylene group, and R₁₆ is a hydrogen atom, a methyl group, an ethyl group or a propyl group.

17. The surface-modified serum albumin-metal porphyrin composite according to claim 16, wherein R₁₄ is a 1,1-disubstituted C₁ - C₁₀ alkane group, a 1-substituted cyclopropyl group, a 1-substituted cyclopentyl group, a 1-substituted cyclohexyl group, or a 2-substituted norbornyl group (the substituent in these groups is a methyl group, an alkylamido group, an alkylester group, or an alkylether group), 1-methyl-2-clyclohexenyl group, or 1-adamantyl group.

18. The surface-modified serum albumin-metal porphyrin composite according to claim 1, wherein the serum albumin clathrating the metal porphyrin is represented by a recombinant human serum albumin-metal porphyrin composite which is obtained by an axial coordination bonding of the metal porphyrin to a recombinant human serum albumin in which at least one amino acid, which brings about a bond with the metal porphyrin, is introduced by a genetic recombination technology.

19. The surface-modified serum albumin-metal porphyrin composite according to claim 6, wherein M of the metal porphyrin is Fe or Co.

20. The surface-modified serum albumin-metal porphyrin composite according to claim 9, wherein M of the metal porphyrin is Fe or Co.

21. The surface-modified serum albumin-metal porphyrin composite according to claim 12, wherein M of the metal porphyrin is Fe or Co.

22. The surface-modified serum albumin-metal porphyrin composite according to claim 14, wherein M of the metal porphyrin is Fe or Co.

23. The surface-modified serum albumin-metal porphyrin composite according to claim 17, wherein M of the metal porphyrin is Fe or Co.

24. The surface-modified serum albumin-metal porphyrin composite according to claim 18, wherein the metal of the metal porphyrin is Fe or Co.

25. The surface-modified serum albumin-metal porphyrin composite according to claim 19, wherein a valence number of Fe is positive bivalent or positive trivalent.

26. The surface-modified serum albumin-metal porphyrin composite according to claim 20, wherein a valence number of Fe is positive bivalent or positive trivalent.

27. The surface-modified serum albumin-metal porphyrin composite according to claim 21, wherein a valence number of Fe is positive bivalent or positive trivalent.

28. The surface-modified serum albumin-metal porphyrin composite according to claim 22, wherein a valence number of Fe is positive bivalent or positive trivalent.

29. The surface-modified serum albumin-metal porphyrin composite according to claim 23, wherein a valence number of Fe is positive bivalent or positive trivalent.

30. The surface-modified serum albumin-metal porphyrin composite according to claim 24, wherein a valence number of Fe is positive bivalent or positive trivalent.

31. The surface-modified serum albumin-metal porphyrin composite according to claim 19, wherein a valence number of Co is positive bivalent.

32. The surface-modified serum albumin-metal porphyrin composite according to claim 20, wherein a valence number of Co is positive bivalent.

33. The surface-modified serum albumin-metal porphyrin composite according to claim 21, wherein a valence number of Co is positive bivalent.

34. The surface-modified serum albumin-metal porphyrin composite according to claim 22, wherein a valence number of Co is positive bivalent.

35. The surface-modified serum albumin-metal porphyrin composite according to claim 23, wherein a valence number of Co is positive bivalent.

36. The surface-modified serum albumin-metal porphyrin composite according to claim 24, wherein a valence number of Co is positive bivalent.

37. The surface-modified serum albumin-metal porphyrin composite according to claim 1, wherein albumin is human serum albumin, bovine serum albumin, recombinant human serum albumin, and albumin multimer.

38. A surface-modified serum albumin-metal porphyrin composite according to claim 1, wherein the number of the polyoxyethylene bonded per one molecule of the albumin is 1 to 15 in average.

39. The surface-modified serum albumin-metal porphyrin composite according to claim 1, wherein an average molecular weight of the polyoxyethylene is 1,000 to 20,000.

40. An oxygen infusion solution which contains the surface-modified serum albumin-metal porphyrin composite according to claim 37.

41. An oxygen infusion solution which contains the surface-modified serum albumin-metal porphyrin composite according to claim 38.

42. An oxygen infusion solution which contains the surface-modified serum albumin-metal porphyrin composite according to claim 39.
